# EUROPÄISCHE PATENTANMELDUNG

(11) **EP 1 275 406 A2**
(43) Veröffentlichungstag der Anmeldung: **15.01.2003**
(21) Anmeldenummer: 02013896.2
(22) Anmeldetag: 24.06.2002
(51) Int. Cl.: A61M 1/00, A61B 17/32

(54) **Gehäuse für einen Vibrationsantrieb**

(30) Priorität: 27.06.2001 DE 20110809 U
(71) Anmelder: Möller Feinmechanik GmbH & Co. KG, 36043 Fulda (DE)
(72) Erfinder: Schröter, Werner, 36137 Grossenlüder (DE)
(74) Vertreter: Hebing, Norbert

(57) **Zusammenfassung**

Die Erfindung bezieht sich auf ein Gehäuse (1) für einen Vibrationsantrieb, der z.B. benötigt wird, um eine Fettabsaugkanüle (8) in Längsschwingungen zu versetzen.

Ein solches Gehäuse (1) soll ergonomisch geformt sein, eine sichere Aufnahme für den Vibrationsantrieb schaffen und leicht zu reinigen sein.

Es wird daher vorgeschlagen, dass das Gehäuse (1) einen zylinderförmigen Kopfbereich (2) zur Aufnahme des Vibrationsantriebs und einen sich daran anschließenden, langgestreckten, im Schnitt halbkreisförmigen Griffbereich (3) aufweist, wobei der vom Bogen des Halbkreises geformte Abschnitt des Griffbereichs (3) bündig in den Kopfbereich (2) übergeht, der von der Sekante des Halbkreises geformte Abschnitt eine Fingerablagefläche (10) bildet und im Bogenabschnitt mindestens eine Daumenmulde (13) ausgeformt ist.

## Beschreibung

Die Erfindung bezieht sich auf ein Gehäuse für einen Vibrationsantrieb, der z.B. benötigt wird, um eine Fettabsaugkanüle in Längsschwingungen zu versetzen, damit sie leichter in das abzusaugende Körperfett eingeführt werden kann.

Aus der EP 0 509 131 A1 ist ein Fettabsaugegerät bekannt, bei dem ein Griffstück mit einem Ultraschallerzeuger verbunden ist. Nachteilig dabei ist, dass mit dieser Anordnung nicht nur die Kanüle in Schwingung versetzt wird, sondern auch das Griffstück und damit die Hand des Operateurs, was zu deutlichen Belastungen der Hand und Ermüdungserscheinungen führt.

Die Erfindung beruht daher auf der Aufgabe, einen Vibrationsantrieb zu schaffen, bei dem die erzeugten Vibrationen vor allem auf das angeschlossene Gerät, z.B. eine Fettabsaugkanüle, übertragen werden und nicht auf die Hand desjenigen, der das Gerät bedient. Die Erfindung beruht vor allem auf der Aufgabe, ein Gehäuse für einen solchen Vibrationsantrieb zu schaffen, das leicht und bequem in der Hand liegt und eine den Operateur nicht belastende Handhabung einer Fettabsaugkanüle ermöglicht.

Die Aufgabe wird mit einem Gehäuse für einen Vibrationsantrieb gelöst, das aus einem zylinderförmigen Kopfbereich zur Aufnahme eines Motors zum Antrieb einer vorne aus dem Gehäuse hervorstehenden Geräteaufnahme und einem sich an den Kopfbereich nach hinten anschließenden, langgestreckten, im Schnitt halbkreisförmigen Griffbereich besteht, wobei der vom Bogen des Halbkreises geformte Abschnitt des Griffbereichs bündig in den Kopfbereich übergeht, der von der Sekante des Halbkreises geformte Abschnitt eine Fingerablagefläche bildet und im Bogenabschnitt mindestens eine Daumenablage ausgebildet ist.

Der Grundgedanke der Überlegung besteht darin, dass der Vibrationsantrieb in einem als Griff ausgeformten Gehäuse angeordnet ist, wobei der Griffbereich des Gehäuses sich unmittelbar an den Motoraufnahmebereich im Kopfbereich des Gehäuses anschließt, so dass das Gewicht des Antriebsmotors nahe bei der Griffhand liegt. Dies hat zur Folge, dass das Gewicht des Motors keine allzu großen Drehmomente auf die Hand ausübt, wodurch der Vibrationsantrieb ermüdungsfrei und sicher geführt werden kann. Da der Griffbereich nicht rotationssymmetrisch ist, ist die Orientierung der Geräteaufnahme zur Hand klar definiert. Die Daumenablagen sind dabei vorzugsweise in Form von Daumenmulden ausgebildet und verbessern dadurch zusätzlich die Führung des Vibrationsantriebes durch den Operateur.

Vorzugsweise sind zwei symmetrisch zueinander angeordnete Daumenmulden an der Außenseite des Gehäuses ausgeformt, so dass das Gehäuse sowohl mit der rechten als auch mit der linken Hand gegriffen werden kann.

Der Griff sollte es weiterhin ermöglichen, dass vor allem Kräfte in Längsrichtung ausgeübt werden. Das ist z.B. dann der Fall, wenn an der Geräteaufnahme eine Fettabsaugkanüle befestigt ist, die in das Körperfett eingeführt werden muss. Damit das Gehäuse entsprechende Druckkräfte aufnehmen kann, ist der Abschnitt zwischen der Fingerablagefläche und dem Kopfbereich durch eine Gehäusewand geschlossen, die als Gegenfläche für den auf der Fingerablagefläche liegenden Zeigefinger dient. Der Operateur kann somit beim Einführen der Fettabsaugkanüle in das Körperfett feinfühlig eine ausreichende Kraft auf den Vibrationsantrieb und damit auf die Fettabsaugkanüle ausüben.

Um bei aller Einfachheit des Gehäuses eine möglichst ergonomische Form zu erhalten, ist vorgesehen, dass die Fingerablagefläche leicht nach außen gekrümmt ist. Dies entspricht der natürlichen Haltung der Finger. Eine weitere Anpassung an eine bestimmte Finger- oder Handform scheint nicht notwendig zu sein, da der Griff in aller Regel von verschiedenen Personen in Benutzung genommen wird.

Eine weitere ergonomische Anpassung ergibt sich dadurch, dass sich der Griffbereich nach hinten zur Achse des Kopfbereiches hin verjüngt. Damit ergibt sich eine leicht in der Hand liegende ästhetische Form. Außerdem wird durch diese Form die Kraftausübung beim Einführen der Kanüle in das Körperfett erleichtert.

Um den Antriebsmotor in das Gehäuse einsetzen zu können, weist der Kopfbereich eine vordere Öffnung auf, die durch eine abnehmbare Kappe geschlossen ist. Die Öffnung hat im Wesentlichen den Durchmesser des Kopfbereiches. Nach Abnehmen der Kappe ist der Kopfbereich offen und der Motor kann leicht eingesetzt oder zu Reparatur- oder Reinigungszwecken entnommen werden.

Vorzugsweise handelt es sich bei dem Motor um einen Elektromotor, dessen Stromversorgung über ein Kabel erfolgt, das durch den Griffbereich verläuft und durch eine Öffnung mit einer Tülle am hinteren Ende des Griffbereiches hindurchgeführt ist. Auf diese Weise ist das Kabel zur Stromversorgung des Motors im Gehäuse verlegt und tritt hinter der Hand des Operateurs aus dem Gehäuse hervor und behindert damit nicht die Führung des Vibrationsantriebes.

An der Tüllenbefestigung ist weiterhin ein Halter vorgesehen, in dem ein Schlauch befestigt werden kann, der mit einer an der Geräteaufnahme befestigten Fettabsaugkanüle verbindbar ist. Dieser Schlauch ist in der Regel durchsichtig, so dass der Operateur den Fortschritt bei der Fettabsaugung durch die Menge des durch den Schlauch fließenden Fettes unter Kontrolle behält.

Da der Vibrationsantrieb bei chirurgischen Handlungen eingesetzt wird, ist es notwendig, ihn nach jedem Einsatz zu sterilisieren. Das erfolgt in der Regel mittels Heißdampf. Dies setzt voraus, dass das Gehäuse den dabei auftretenden Temperaturen widersteht. Außerdem ist es notwendig, dass die Öffnungen im Gehäuse, insbesondere die Durchführungen für die Geräteaufnahme und des Kabels, abgedichtet sind. Auch die dafür vorgesehenen Dichtungen müssen resistent gegenüber einer Heißdampfsterilisation sein.

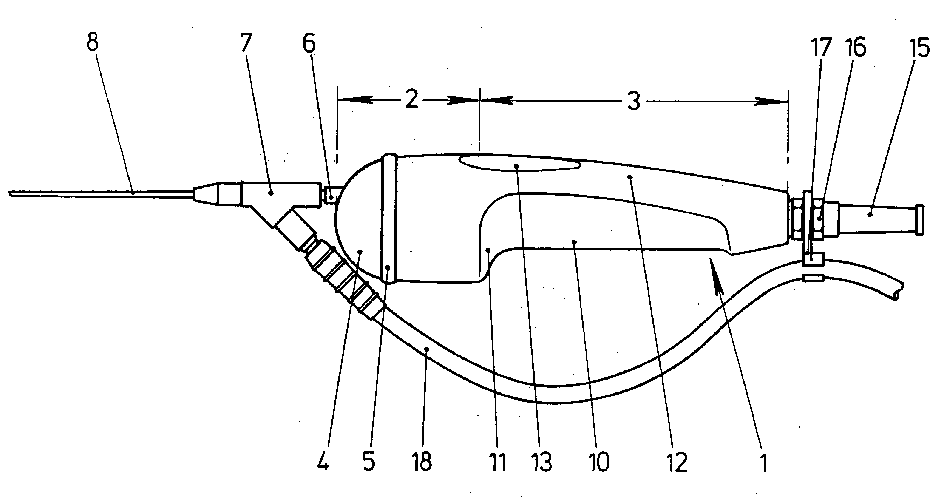

Im Folgenden wird anhand eines Ausführungsbeispiels die Erfindung näher erläutert. Dazu zeigt die einzige Figur eine perspektivische Darstellung des Gehäuses für einen Vibrationsantrieb.

Das Gehäuse 1 hat eine langgestreckte Form mit einem Kopfbereich 2 und einem Griffbereich 3. Bei dem Kopfbereich handelt es sich um einen zylinderförmigen Abschnitt, der vorne durch ein Kappe 4 geschlossen ist, die mittels einer Überwurfmutter 5 am zylinderförmigen Abschnitt befestigt ist. Im Kopfbereich liegt ein in der Darstellung nicht zu erkennender Elektromotor, der über ein Getriebe auf eine Geräteaufnahme 6 eine hin- und hergehende Bewegung ausübt. Der Hub liegt in der Regel bei ca. 2,8 mm, die Hubgeschwindigkeit bei 2.000 bis 6.000 Hüben pro Minute, die mittels eines elektronischen Reglers eingestellt wird, der ebenfalls im Gehäuse 1 untergebracht werden kann.

Die Geräteaufnahme 6 ist durch eine Öffnung in der Kappe 4 nach vorne herausgeführt. In diesem Ausführungsbeispiel ist sie mit dem Kopfstück 7 einer Fettabsaugkanüle 8 verbunden.

Bei dem Griffbereich 3 handelt es sich um eine konisch nach hinten zulaufende Verlängerung des zylindrischen Abschnittes des Kopfstückes 7, wobei allerdings eine Seite nach innen eingezogen ist, so dass der Griffbereich 3 im Schnitt einen Halbkreis bildet. Die Sekante dieses Halbkreises formt die leicht nach außen gerundete Fingerablagefläche 10. Auf der gegenüberliegenden runden Seite des Griffbereiches 3 liegt der Handballen auf. Der Übergang des Kopfbereiches 2 in die Fingerablagefläche 10 ist durch eine Wand geschlossen, die als Gegenfläche 11 für den Zeigefinger dient.

Auf der runden Oberseite 12 des Griffbereiches 3 sind zwei Daumenmulden 13 angeordnet, von denen im Bild eine zu sehen ist. Die andere liegt symmetrisch zu jener auf der gegenüberliegenden, nicht zu erkennenden Seite des Gehäuses. Wird das Gehäuse 1 von einem Rechtshänder ergriffen, so liegt der Daumen in der dargestellten Daumenmulde 13, bei einem Linkshänder wird die nicht zu erkennende Daumenmulde genutzt.

Im verjüngten Ende des Gehäuses 1 ist eine Tülle 15 an einer aus dem Gehäuse 1 herausragenden Hohlschraube 16 befestigt. Die Tülle 15 wird aufgeschraubt und erhält dabei gleichzeitig einen Halter 17 für einen Schlauch 18, der mit dem Kopfstück 7 der Fettabsaugkanüle 8 verbunden ist. Durch diesen Schlauch 18 wird das abgesaugte Fett zu einem hier nicht dargestellten Behälter geleitet.

### Bezugszeichenliste

- 1: Gehäuse
- 2: Kopfbereich
- 3: Griffbereich
- 4: Kappe
- 5: Überwurfmutter

- 6: Geräteaufnahme
- 7: Kopfstück
- 8: Fettabsaugkanüle
- 10: Fingerablagefläche

- 11: Gegenfläche
- 12: Oberseite
- 13: Daumenmulde
- 15: Tülle

- 16: Hohlschraube
- 17: Halter
- 18: Schlauch

## Patentansprüche

1. Gehäuse für einen Vibrationsantrieb, **dadurch gekennzeichnet, dass** es aus einem zylinderförmigen Kopfbereich (2) zur Aufnahme eines Motors zum Antrieb einer vorne aus dem Gehäuse (1) hervorstehenden Geräteaufnahme (6) und mit einem sich an den Kopfbereich (2) nach hinten anschließenden, langgestreckten, im Schnitt halbkreisförmigen Griffbereich (3) besteht, wobei der vom Bogen des Halbkreises geformte Abschnitt des Griffbereichs (3) bündig in den Kopfbereich (2) übergeht, der von der Sekante des Halbkreises geformte Abschnitt eine Fingerablagefläche (10) bildet und im Bogenabschnitt mindestens eine Daumenablage ausgebildet ist.

2. Gehäuse nach Anspruch 1, **dadurch gekennzeichnet, dass** zwei symmetrisch zueinander angeordnete, die Daumenablage bildenden Daumenmulden (13) ausgeformt sind, so dass das Gehäuse (1) sowohl mit der rechten als auch mit der linken Hand gegriffen werden kann.

3. Gehäuse nach Anspruch 1 oder 2, **dadurch gekennzeichnet, dass** der Abschnitt zwischen der Fingerablagefläche (10) und dem Kopfbereich (2) durch eine Gehäusewand geschlossen ist, die als Gegenfläche (11) für den auf der Fingerablagefläche (10) liegenden Zeigefinger dient.

4. Gehäuse nach einem der vorhergehenden Ansprüche, **dadurch gekennzeichnet, dass** die Fingerablagefäche (10) leicht nach außen gekrümmt ist.

5. Gehäuse nach einem der vorhergehenden Ansprüche, **dadurch gekennzeichnet, dass** sich der Griffbereich (3) nach hinten zur Achse des Kopfbereiches (2) hin verjüngt.

6. Gehäuse nach einem der vorhergehenden Ansprüche, **dadurch gekennzeichnet, dass** der Kopfbereich (2) eine vordere Öffnung aufweist, die durch eine abnehmbare Kappe (4) geschlossen ist.

7. Gehäuse nach Anspruch 5, **dadurch gekennzeichnet, dass** der Motor ein Elektromotor ist, dass am hinteren Ende des Gehäuses (1) an einer Öffnung eine Tülle (15) befestigt ist und dass ein Motorstromkabel durch die Tülle und den Griffbereich (3) zum Elektromotor geführt ist.

8. Gehäuse nach Anspruch 6, **dadurch gekennzeichnet, dass** sich an der Befestigung der Tülle (15) ein Halter (17) für einen Schlauch (18) befindet, der mit einer an der Geräteaufnahme (6) befestigten Fettabsaugkanüle (8) verbindbar ist.

9. Gehäuse nach einem der vorhergehenden Ansprüche, **dadurch gekennzeichnet, dass** alle Öffnungen im Gehäuse (1) durch Dichtungen abgedichtet sind, die einer sterilisierenden Reinigung mittels Heißdampf widerstehen.
